# FASCICULE DE BREVET EUROPEEN

(11) **EP 1 090 629 B1**
(45) Date de publication et mention de la délivrance du brevet: **04.08.2004**
(21) Numéro de dépôt: 00402462.6
(22) Date de dépôt: 07.09.2000
(51) Int. Cl.: A61K 7/48

(54) **Association d'escine et de sulfate de dextran**
Kombination aus Aescin und Dextran Sulphat
Aescin and dextran sulfate association

(30) Priorité: 08.10.1999 FR 9912589
(43) Date de publication de la demande: 11.04.2001
(73) Titulaire: L'OREAL, 75008 Paris (FR)
(72) Inventeur: Renault Béatrice, 94410 Saint-Maurice (FR)
(74) Mandataire: Galup, Cédric Olivier Nicolas

(56) Documents cités:
- EP-A- 0 803 246
- FR-A- 2 047 874
- FR-A- 2 668 061
- FR-A- 2 755 012

## Description

L'invention a pour objet une composition comprenant, à titre d'agent actif, dans un milieu physiologiquement acceptable, l'association de sulfate de dextran et d'escine, son utilisation, ainsi qu'un procédé de traitement cosmétique des rougeurs et/ou des poches et/ou des cernes péri-oculaires.

Le corps en général est sensible aux agressions externes de la vie moderne (exposition aux UV, variations importantes d'hygrométrie et de température, pollution, etc ... ). La réponse à ces agressions peut se traduire entre autres par l'apparition de rougeurs liées à une vasodilatation locale ou encore par l'apparition d'oedèmes locaux.

Dans le domaine des désordres cutanés, il est connu que certaines peaux sont plus sensibles que d'autres aux agressions externes.
La demanderesse a pu déterminer que les symptômes liés aux peaux sensibles. sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc., en réponse à différents facteurs tels que l'environnement, les émotions, les aliments, le vent, les frottements, le rasoir, le savon, les tensioactifs, l'eau dure à forte concentration de calcaire, les variations de température ou la laine.

Le contour de l'oeil, de par sa structure et sa forte innervation, représente un site anatomique particulièrement sensible aux agressions ou aux stress mécaniques comportementaux (frottements). Suite à ces stimuli et en raison d'un seuil interne très bas de sensibilité neurogène chez certains individus plus sensibles, le contour de l'oeil témoigne rapidement par des rougeurs et/ou des démangeaisons, conséquences d'une vasodilatation exacerbée, d'un inconfort passager mais aussi par des poches et/ou des cernes et/ou un oedème, d'inconfort plus persistant traduisant un épuisement métabolique plus important de l'épiderme et du derme.

Outre ces données physiologiques, on sait que du simple aspect cosmétique les rougeurs, les cernes et les poches ont toujours été considérées comme inesthétiques et que l'on a toujours cherché à les masquer voire à les éliminer.

A cet égard la demanderesse vient maintenant de découvrir que l'association de sulfate de dextran et d'escine présente de remarquables propriétés apaisantes et/ou inhibitrices de la vasodilatation et/ou anti-oedémateuse, et ce dans des proportions qui dépassent le simple cumul des effets obtenus avec chacun des composants pris individuellement.

Le dextran est un polysaccharide neutre sans groupe chargé, biologiquement inerte, préparé par fermentation du sucre de betterave contenant uniquement des groupements hydroxyles. Il est possible d'obtenir à partir du dextran natif par hydrolyse et purification des fractions de dextran de poids moléculaires différents. Le dextran peut se présenter sous la forme de sulfate de dextran.

Outre les propriétés physico-chimiques du sulfate de dextran qui sont connues de l'art antérieur et qui en font un bon composé pour des compositions cosmétiques (bonne solubilité dans l'eau et les solutions salines, grande stabilité dans des solutions de pH allant de 4 à 10 à température ambiante), il est également décrit que le sulfate de dextran possède des propriétés d'absorption d'eau, d'effet protecteur contre les dommages induits par les radicaux libres, particulièrement en application topique, de stabilisation des protéines ou substances instables, d'hydratation du fait de ses excellentes propriétés hydrophiles. Des propriétés biologiques telles qu'un effet anticoagulant, un effet inhibiteur d'enzyme comme l'hyaluronidase, les glucosidases, l'élastase ou encore la thrombine, une activité antivirale sont également décrites. Au niveau de la peau et de la protection de la peau, le sulfate de dextran est connu pour ses propriétés antirides, anti-inflammatoire, anti-allergique, anti-âge, pour son rôle dans le traitement de la peau rugueuse et écailleuse et dans l'hydratation.

L'escine est une molécule chimique constituée d'un acide glucuronique et de deux sucres (glucose-xylose) liés à un aglycone, la desglucoescine qui a un poids moléculaire de 1131,24. C'est une molécule que l'on retrouve par exemple dans des extraits végétaux, particulièrement dans les extraits de marron d'inde. Dans l'art antérieur l'escine est décrite dans des compositions amincissantes (FR-2729856, EP-034153, WO-9742928, WO-9815259), dans des compositions favorisant la circulation sanguine (EP-158090, US-4983626), dans des compositions destinées au traitement de la peau comme anti-inflammatoire (EP-728472), pour améliorer la cohésion entre le derme et l'épiderme (WO-9819664), dans des compositions cosmétiques éclaircissantes (JP-07076512).

Il est également connu dans l'art antérieur l'utilisation de l'escine dans des compositions destinées au traitement des poches et des rides localisées sous les yeux (FR-2668061, US-5273916, US-5571503).

Cependant, à la connaissance de la demanderesse l'effet surprenant de synergie qu'elle a mis en évidence pour l'association de l'escine et du sulfate de dextran sur l'inhibition de la vasodilatation et/ou l'effet anti-oedémateux et/ou l'apaisement des peaux sensibles n'a jamais été décrit dans l'art antérieur.

Ainsi, la présente invention a pour objet premier une composition comprenant, à titre d'agent actif, dans un milieu physiologiquement acceptable, l'association de sulfate de dextran et d'escine.

Par milieu physiologiquement acceptable, on comprend un milieu compatible avec la peau, les muqueuses, les ongles, les cheveux.

Préférentiellement selon l'invention, la composition comprend du sulfate de dextran, sous la forme d'un sel de sodium.

Selon l'invention, le sulfate de dextran a un poids moléculaire compris entre 2.10³ et 5.10⁶ et préférentiellement compris entre 5.10³ et 10⁵.

Bien entendu, le sulfate de dextran peut être de toute origine. Préférentiellement la composition de l'invention comprend du sulfate de dextran vendu par la société Pharmacia Biotech sous la dénomination Dextran sulfate 10 sodium salt®.

La composition de l'invention comprend de l'escine qui peut être d'origine synthétique ou naturelle.
Par origine synthétique, on entend de l'escine, à l'état pur ou en solution quelle qu'en soit sa concentration dans ladite solution, obtenu par synthèse chimique. Par origine naturelle, on entend de l'escine, à l'état pur ou en solution quelle qu'en soit sa concentration dans ladite solution, obtenu à partir d'un élément naturel comme par exemple un extrait végétal, particulièrement un extrait de marron d'inde.

Selon l'invention, la composition comprend préférentiellement de l'escine vendue par la société Indena sous la dénomination commerciale escine 3030000® ou encore de l'escine vendue par la société LEK sous la dénomination commerciale amorphous beta Aescin®.

Les quantités de sulfate de dextran et d'escine utilisables dans la composition de l'invention dépendent bien évidemment de l'effet recherché.

A titre d'exemple la quantité pondérale de sulfate de dextran utilisable dans la composition de l'invention peut aller par exemple de 0,01% à 5% et de préférence de 0,05% à 2% du poids total de la composition.

Pour donner un ordre de grandeur, la quantité pondérale d'escine utilisable selon l'invention représente de 0,005% à 5% du poids total de la composition, préférentiellement de 0,01% à 2% du poids total de la composition.

Dans la composition de l'invention, le rapport pondéral entre le sulfate de dextran et l'escine est compris entre 2.10⁻³ et 10³ et de préférence compris entre 25.10⁻³ et 200.

Bien qu'elle puisse être utilisée dans toutes formes d'application, la composition de l'invention est préférentiellement destinée à une application topique.

La composition de l'invention peut être une composition cosmétique ou dermatologique. Préférentiellement selon l'invention, la composition est une composition cosmétique et encore plus préférentiellement une composition cosmétique d'application topique.

L'invention a en outre pour objet l'utilisation de l'association de sulfate de dextrane et d'escine pour fabriquer une composition destinée à traiter, de manière préventive ou curative, oedèmes cutanés et/ou les peaux sensibles et/ou les picotements et/ou les fourmillements et/ou les prurits et/ou les tiraillements et/ou les échauffements et/ou les érythèmes.

Les signes cliniques de la peau sensible sont en effet essentiellement subjectifs : picotements, fourmillements, prurits, tiraillements, échauffements, et ils s'associent parfois à des érythèmes.

On a vu précédemment dans le texte que la zone péri-oculaire est une zone particulièrement sensible aux agressions externes.
Ainsi, l'invention a aussi pour objet. l'utilisation cosmétique dans une composition de l'association de sulfate de dextran et d'escine pour traiter, de manière préventive ou curative, les rougeurs, les poches et/ou les cernes péri-oculaires.

La composition selon l'invention ou celle utilisée selon l'invention, comprend bien évidemment un support cosmétiquement acceptable, c'est à dire un support compatible avec la peau, les muqueuses, les ongles, les cheveux et peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que les nanosphères et les nanocapsules ou mieux des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick. Elle peut être utilisée comme produit de soin, comme produit de nettoyage, comme produit de maquillage ou encore comme simple produit déodorant. De façon connue, la composition de l'invention peut contenir également les adjuvants habituels dans les domaines cosmétiques et dermatologique, tels que les gélifiants hydrophiles ou lipophiles, les actifs hydrophiles ou lipophiles, les conservateurs, les antioxydants, les solvants, les parfums, les charges, les filtres, les pigments, les agents chélateurs, les absorbeurs d'odeur et les matières colorantes. Les quantités de ces différents adjuvants sont celles classiquement utilisées dans les domaines considérés, et par exemple de 0,01% à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition de l'invention est une émulsion, la proportion de la phase grasse peut aller de 5 % à 80 % en poids, et de préférence de 5 % à 50 % du poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 % à 30 % en poids, et de préférence de 0,5 % à 20 % du poids total de la composition.

Comme huiles utilisables dans l'invention, on peut citer les huiles minérales, les huiles d'origine végétale (huile d'abricot, huile de tournesol), les huiles d'origine animale, les huiles de synthèse, les huiles siliconées et les huiles fluorées (perfluoropolyéthers). On peut aussi utiliser comme matières grasses des alcools gras (alcool cétylique), des acides gras, des cires (cire d'abeilles).

Comme émulsionnants et coémulsionnants utilisables dans l'invention, on peut citer par exemple les esters d'acide gras et de polyéthylène glycol tels que le stéarate de PEG-40, le stéarate de PEG-100, les esters d'acide gras et de polyol tels que le stéarate de glycéryle et le tristéarate de sorbitane.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxy-acides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Il est également possible d'utiliser dans la composition de l'invention, en plus de l'association selon l'invention, des composés choisis parmi :
- les hormones végétales ;
- les agents antagonistes de calcium, comme le vérapamil et le Diltiazem ;
- des capteurs de radicaux OH, tels que le diméthylsulfoxyde ;
- des ouvreurs de canaux chlore ;
- des extraits de végétaux tels que ceux d'Iridacées, de Rosacées ou de soja, extraits pouvant alors contenir ou non des isoflavones ;
- des extraits de micro-organismes dont en particulier des extraits bactériens comme ceux de bactéries filamenteuses non photosynthétiques.

A la liste ci-dessus, d'autres composés peuvent également être rajoutés, à savoir par exemple les ouvreurs de canaux potassiques tels que le diazoxyde et le minoxidil, la spiroxazone, des phospholipides comme la lécithine, les acides linoléique et linolénique, l'acide salicylique et ses dérivés décrits dans le brevet français FR 2 581 542, comme les dérivés de l'acide salicylique porteurs d'un groupement alcanoyle ayant de 2 à 12 atomes de carbone en position 5 du cycle benzénique, des acides hydroxycarboxyliques ou cétocarboxyliques et leurs esters, des lactones et leurs sels correspondants, l'anthraline, des caroténoïdes, les acides eicosatétraénoïque et eicosatriénoïque ou leurs esters et amides, la vitamine D et ses dérivés.

Selon l'invention, on peut ajouter dans la composition selon l'invention d'autres agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les agents modulant l'adhésion bactérienne sur la peau et /ou les muqueuses tels que le miel, notamment le miel d'acacias et certains dérivés de sucres ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxy-acides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les anti-séborrhéiques tels que la progestérone ;
- les antipelliculaires comme l'octopirox ou la pyrithione de zinc ;
- les antiacnéiques comme l'acide rétinoïque ou le peroxyde de benzoyle.
- des substances telles que les antagonistes de substance P, de CGRP ou de bradykinine ou les inhibiteurs de NO synthase ou encore les inhibiteurs de canaux sodiques, composés décrits comme étant actifs dans le traitement des peaux sensibles et comme présentant des effets anti-irritants, en particulier vis-à-vis de composés irritants éventuellement présents dans les compositions ;
- des agents capables de moduler la réponse allergique de la peau telles que le LPV.

Comme actifs, on peut utiliser notamment les hydratants tels que les polyols (par exemple la glycérine), les vitamines (par exemple le D-panthénol), les agents anti-inflammatoires, les agents apaisants (allantoïne, eau de bleuet), les filtres UVA et UVB, les agents matifiants (par exemple les polydiméthylorganosiloxanes partiellement réticulés vendus sous le nom KSG® par Shin Etsu) et leurs mélanges.

On peut aussi ajouter des actifs antirides, et notamment des produits tenseurs tels que les protéines végétales et leurs hydrolysats, en particulier l'extrait de protéines de soja vendu sous le nom d'Eleseryl® par la société LSN ou le dérivé d'avoine vendu sous la dénomination Reductine® par la société Silab.

L'invention a en outre pour objet un procédé de traitement cosmétique des rougeurs, des poches et des cernes péri-oculaires, caractérisé par le fait que l'on applique sur la peau une composition cosmétique comprenant au moins l'association du sulfate de dextran et de l'escine.

Le procédé de l'invention est un procédé cosmétique dans la mesure où il a pour but d'améliorer l'aspect esthétique de l'individu.

Le procédé de traitement cosmétique de l'invention peut être mis en oeuvre notamment en appliquant les compositions cosmétiques telles que définies ci-dessus, selon la technique d'utilisation habituelle de ces compositions. Par exemple : application de crèmes, de gels, de sérums, de lotions, de laits, de compositions anti-solaires, sur la peau.

Les exemples et compositions suivants illustrent l'invention sans la limiter aucunement. Dans les compositions les proportions indiquées sont des pourcentages en poids.

### Exemple 1 : Etude de l'effet anti-oedémateux et l'effet inhibiteur de la vasodilatation de l'association du sulfate de dextran et de l'escine :

Le but de cette étude est de visualiser l'effet anti-oedémateux et l'effet inhibiteur de la vasodilatation de l'association du sulfate de dextran et de l'escine, dans un modèle de peau maintenue en survie (paupière) après induction de l'oedème et de la vasodilatation par une association neuromédiateur (substance P) / facteur de nécrose tumorale de type α (TNFα) / acide arachidonique.

Les comparaisons suivantes sont réalisées :
- Peau témoin : peau sans aucun traitement ;
- Peau soumise à l'action de la substance P, du TNFα et de l'acide arachidonique ;
- Peau soumise à l'action de la substance P, du TNFα, de l'acide arachidonique et traitée à l'escine ;
- Peau soumise à l'action de la substance P, du TNFα, de l'acide arachidonique et traitée au sulfate de dextran ;
- Peau soumise à l'action de la substance P, du TNFα, de l'acide arachidonique et traitée par l'association sulfate de dextran / escine.

### A) Matériel et méthodes

### 1) Maintien en survie des fragments de peau humaine

Des fragments de peau humaine normale provenant de 3 donneurs différents (chirurgie plastique) et mécaniquement débarrassés de la graisse sous-cutanée et de la couche inférieure du derme, sont déposés chacun sur un insert de culture constitué d'une membrane poreuse (pores de diamètre 12 µm) (Costar) la partie dermique du fragment de peau étant disposée au contact de la membrane poreuse. Le montage ainsi réalisé est positionné sur les puits d'une plaque de culture 12 puits de marque Costar et laissé 24 heures à 37°C dans un incubateur en atmosphère humidifiée contenant 5% de CO₂.
Du milieu de culture DMEM (Gibco-BRL) contenant 100 U/ml de pénicilline et 100 µg/ml de streptomycine, 200 µg/ml de L-glutamine et 0,1% de sérum de veau foetal (D.A.P., France), est ajouté dans le fond des puits de sorte que le liquide atteigne la membrane poreuse sans la dépasser. Ainsi disposée, la peau est en contact par sa face inférieure au travers de la membrane poreuse avec le milieu de culture, un passage s'effectuant par diffusion lente entre les deux compartiments.

### 2) Induction de l'oedème et de la dilatation des capillaires par l'association d'un neuromédiateur, du TNFα et d'acide arachidonique

L'oedème et la vasodilatation sont réalisés expérimentalement en déposant à la surface de la peau un mélange dans du milieu de culture constitué de 25µl d'un neuromédiateur (Substance P à 5 µM), d'acide arachidonique (25 µl d'une solution à 40 mg/ml dans du glycérol) et de TNFα (50 ng/ml), par ailleurs appelé "cocktail A" . Ces substances sont maintenues 2 heures au contact de la peau.

### 3) Application des produits à tester

Les produits à tester sont appliqués de manière topique sur les fragments de peau pendant l'induction de l'oedème et de la vasodilatation.
L'escine, à la concentration finale de 0,075%, est solubilisée dans l'eau.
Le sulfate de dextran, à la concentration finale de 0,20 %, est solubilisé dans le propylène glycol.
La température est maintenue à 37°C durant tout le temps de l'essai.

### 4) Analyse histologique

Les analyses histologiques sont réalisées selon les techniques classiques connues de l'art antérieur sans modifications des protocoles.
Après traitement, les fragments de peau sont prélevés et fixés dans le liquide de Bouin et inclus en paraffine en vue des analyses histologiques.

Après coloration par l'hémalun-éosine, les 2 critères, oedème et dilatation des capillaires, sont évalués à l'aide de scores histologiques semi-quantitatifs.
L'évaluation histologique est effectuée dans le derme superficiel et dans la partie haute du derme moyen sur un même nombre de champs (4 champs au grossissement 10).
Lorsque l'aspect des capillaires ou de l'oedème est identique sur toute la coupe, un seul score est attribué (exemple : score 1).
Si deux aspects sont notés sur une coupe, un score intermédiaire est attribué (exemple: un score de 1 à 2 est équivalent à un score de 1,5).
Si l'aspect n'est pas uniforme, une évaluation est réalisée champ par champ et une moyenne de tous les champs est effectuée.

### a) Evaluation histologique de l'oedème

| Appréciation | Score |
|---|---|
| pas d'oedème | 0 |
| oedème très léger | 1 |
| oedème modéré | 2 |
| oedème important | 3 |
| oedème très important | 4 |

### b) Evaluation histologique des altérations vasculaires

| Appréciation | Score |
|---|---|
| pas de dilatation | 0 |
| dilatation légère | 1 |
| dilatation modérée | 2 |
| dilatation importante | 3 |
| dilatation très importante | 4 |

### B) Résultats

| | oedème | Vasodilatation |
|---|---|---|
| Peau témoin | 0,62 ± 0,63 | 0,37 ± 0,47 |
| Peau + cocktail A | 2,8 ± 0,76 | 2,2±0,57 |
| Peau + cocktail A + escine | 1, 16 ± 0,77 | 1,5 ± 0,5 |
| Peau + cocktail A + sulfate de dextran | 2,1 ± 1,04 | 1,8 + 1,04 |
| Peau + cocktail A + escine + sulfate de dextran | 0,43 ± 0,51 | 0,5 + 0,5 |

### a) Evaluation de l'oedème

L'application conjointe d'acide arachidonique (25 µl d'une solution à 40 mg/ml dans du glycérol) en topique, de substance P (à 5 µM) et de TNFα (50 ng/ml) dans le milieu de culture permet de générer un oedème.
L'augmentation de cet oedème (score de 2,8) est statistiquement significative (Test apparié de Student, p < 0,05 ) par rapport à la peau témoin pour laquelle un score de 0,62 est obtenu.

L'application d'escine induit une diminution de cet oedème (score de 1,16) diminution statistiquement significative (Test apparié de Student, p < 0,05) par rapport aux produits irritants seuls (cocktail A).

L'application de sulfate de dextran a pour conséquence une tendance à la diminution de cet oedème (score de 2,1). Cette diminution n'est pas statistiquement significative par rapport aux produits irritants du fait de l'importance de l'écart type.

L'application conjointe de escine et du sulfate de dextran génère une diminution de l'oedème (score de 0,43). Les résultats sont statistiquement significatifs (p < 0,05) par rapport aux produits irritants seuls (cocktail A).
Par rapport aux produits appliqués séparément, l'application de l'association produit un effet surprenant de synergie dans l'amélioration du score oedème. Le score obtenu est en effet inférieur aux scores des produits utilisés seuls.

### b) Vasodilatation

L'application topique conjointe d'acide arachidonique (25 µl d'une solution à 40 mg/ml dans du glycérol), de substance P (à 5 µM) et de TNFα (50 ng/ml) dans le milieu de culture permet de générer une dilatation des capillaires au niveau du derme.
L'augmentation des altérations vasculaires (score de 2,2) est statistiquement significative (Test apparié de Student, p < 0,05 ) par rapport aux peaux témoins (score de 0,37).

L'application de l'escine induit une tendance à la diminution de la dilatation des capillaires (score de 1.5). Cette diminution n'est pas statistiquement significative par rapport aux produits irritants seuls

L'application du sulfate de dextran entraîne également une tendance à la diminution de la dilatation des capillaires (score de 1,8), cette diminution n'étant pas statistiquement significative par rapport aux produits irritants seuls.

L'application conjointe des produits génère une diminution de la dilatation des capillaires (score de 0,5).
Les résultats sont statistiquement significatifs par rapport aux produits irritants seuls (l'acide arachidonique, substance P et TNFα).
Par rapport aux produits appliqués séparément, l'application de l'association produit un effet surprenant de synergie dans l'amélioration du score dilatation des capillaires. Le score obtenu est en effet inférieur aux scores des produits utilisés seuls.

### C) Conclusion

Dans le modèle de peau humaine maintenue en survie, l'application conjointe de l'escine et du sulfate de dextran a permis d'observer un effet surprenant de synergie entre les 2 produits avec diminution de l'oedème et de la dilatation vasculaire induits par la substance P, l'acide arachidonique et le TNFα.

### Exemple 2 : Exemples de compositions:

| Composition 1 : composition aqueuse | |
|---|---|
| Escine | 0.50 % |
| Sulfate de dextran | 0.80 % |
| Eau | qsp 100 |

L'eau de cette composition est de l'eau déminéralisée stérile mais elle peut être avantageusement remplacée en totalité ou pour partie par une eau de source ou une eau minérale de type : Eau de la Roche Posay ou Eau de Vichy (source Lucas).

Cette, composition peut être utilisée pure, ou diluée.
Elle peut être introduite sur des lingettes ou des compresses ou des pads stériles afin d'apaiser les paupières.

| Composition 2 : Lingettes apaisantes | |
|---|---|
| Escine | 0,10 % |
| Sulfate de dextran | 0,20 % |
| Eau de bleuet | 1,00 % |
| Glycérol | 3,00 % |
| Eau | qsp 100 |

Cette solution est imprégnée sur des lingettes.
Elle peut également être intégrée dans des patchs décongestionnants.

| Composition 3 : composition concentrée | |
|---|---|
| Glycérine | 30,000 % |
| Acide poly acrylique | 8,000 % |
| Polyacrylate de sodium | 5,500 % |
| Gomme de cellulose | 3,500 % |
| Méthylparaben | 0,160 % |
| Sulfate de dextran | 0,160 % |
| Escine | 0,030 % |
| Eau | qsp 100 |

Cette association peut être introduite dans les gels ou des émulsions eau dans huile ou huile dans eau pour le soin des yeux, anti-poche ou anti-cerne.
Elle peut également être introduite dans des compositions biphasées ou des supports vésiculés de type liposomes ou niosomes.

| Composition 4 : Gel anti-poche | |
|---|---|
| Carbomer | 0,20 % |
| Copolymère acrylamide/acrylamido 2-méthylpropane | |
| sulfonate de sodium/isoparaffine/eau | 2,50 % |
| Escine | 0,05 % |
| Sulfate de dextran | 0,08 % |
| Hydroxyde de sodium | 0,05 % |
| Eau | qsp 100 |

| Composition 5 : émulsion contour des yeux anti-cerne | |
|---|---|
| Vaseline | 4,00 % |
| Tristearate de sorbitan | 0,90 % |
| Myristate de Myristyl | 2,00 % |
| Méthylparaben | 0,25 % |
| Escine | 0,50 % |
| Sulfate de dextran | 1,00 % |
| Eau de La Roche Posay | 10,0 % |
| Eau | qsp 100% |

Cette composition peut également être introduite dans des produits de maquillage contenant des pigments afin d'avoir une activité sur les cernes à la fois immédiate grâce à la présence de pigments et à long terme grâce à l'activité biologique de l'association.

| Composition 6 : émulsion apaisante contour des yeux anti-cerne | |
|---|---|
| Vaseline | 4,00 % |
| Tristearate de sorbitan | 0,90 % |
| Myristate de Myristyl | 2,00 % |
| Méthylparaben | 0,25 % |
| Escine | 0,50 % |
| Sulfate de dextran | 1,00 % |
| Eau de La Roche Posay | 10,0 % |
| Eau | qsp 100% |

## Revendications

1. Composition comprenant, à titre d'agent actif, dans un milieu physiologiquement acceptable, l'association de sulfate de dextran et d'escine.

2. Composition selon la revendication 1, **caractérisée par le fait que** le sulfate de dextran est sous forme de sel de sodium.

3. Composition selon l'une quelconque des revendications précédentes, **caractérisée par le fait que** le sulfate de dextran a un poids moléculaire compris entre 2.10³ et 5.10⁶.

4. Composition selon la revendication précédente, **caractérisée par le fait que** le sulfate de dextran a un poids moléculaire compris entre 5.10³ et 10⁵.

5. Composition selon l'une quelconque des revendications précédentes, **caractérisée par le fait que** le sulfate de dextran est en une quantité pondérale comprise entre 0,01% et 5 % par rapport au poids total de la composition.

6. Composition selon la revendication précédente, **caractérisée par le fait que** le sulfate de dextran est en une quantité pondérale comprise entre 0,05% et 2% par rapport au poids total de la composition.

7. Composition selon l'une quelconque des revendications précédentes, **caractérisée par le fait que** l'escine est en une quantité pondérale comprise entre 0,005% et 5% par rapport au poids total de la composition.

8. Composition selon la revendication précédente, **caractérisée par le fait que** l'escine est en une quantité pondérale comprise entre 0,01% et 2% par rapport au poids total de la composition.

9. Composition selon l'une quelconque des revendications précédentes, **caractérisée par le fait que** le rapport pondéral entre le sulfate de dextran et l'escine est compris entre 2.10⁻³ et 10³.

10. Composition selon la revendication précédente, **caractérisée par le fait que** le rapport pondéral entre le sulfate de dextran et l'escine est compris entre 25.10⁻³ et 200.

11. Utilisation de l'association de sulfate de dextran et d'escine pour fabriquer une composition destinée à traiter, de manière préventive ou curative, les oedèmes cutanés et/ou les peaux sensibles et/ou les picotements et/ou les fourmillements et/ou les prurits et/ou les tiraillements et/ou les échauffements et/ou les érythèmes.

12. Utilisation cosmétique dans une composition de l'association de sulfate de dextran et d'escine pour traiter, de manière préventive ou curative, les rougeurs, les poches et/ou les cernes péri-oculaires.

13. Procédé de traitement cosmétique des rougeurs, des poches et/ou des cemes péri-oculaires, **caractérisé par le fait que** l'on applique sur la peau une composition cosmétique comprenant au moins l'association du sulfate de dextran et de l'escine.

## Patentansprüche

1. Zusammensetzung, die als Wirkstoff in einem physiologisch akzeptablen Medium die Kombination von Dextransulfat und Aescin enthält.

2. Zusammensetzung nach Anspruch 1, **dadurch gekennzeichnet, dass** das Dextransulfat in Form des Natriumsalzes vorliegt.

3. Zusammensetzung nach einem der vorhergehenden Ansprüche, **dadurch gekennzeichnet, dass** das Dextransulfat eine Molmasse von 2·10³ bis 5·10⁶ besitzt.

4. Zusammensetzung nach dem vorhergehenden Anspruch, **dadurch gekennzeichnet, dass** das Dextransulfat eine Molmasse von 5·10³ bis 10⁵ aufweist.

5. Zusammensetzung nach einem der vorhergehenden Ansprüche, **dadurch gekennzeichnet, dass** das Dextransulfat in einer Gewichtsmenge von 0,01 bis 5 %, bezogen auf das Gesamtgewicht der Zusammensetzung, vorliegt.

6. Zusammensetzung nach dem vorhergehenden Anspruch, **dadurch gekennzeichnet, dass** das Dextransulfat in einer Gewichtsmenge von 0,05 bis 2 %, bezogen auf das Gesamtgewicht der Zusammensetzung, vorliegt.

7. Zusammensetzung nach einem der vorhergehenden Ansprüche, **dadurch gekennzeichnet, dass** das Aescin in einer Gewichtsmenge von 0,005 bis 5 %, bezogen auf das Gesamtgewicht der Zusammensetzung, vorliegt.

8. Zusammensetzung nach dem vorhergehenden Anspruch, **dadurch gekennzeichnet, dass** das Aescin in einer Gewichtsmenge von 0,01 bis 2 %, bezogen auf das Gesamtgewicht der Zusammensetzung, vorliegt.

9. Zusammensetzung nach einem der vorhergehenden Ansprüche, **dadurch gekennzeichnet, dass** das Gewichtsverhältnis von Dextransulfat und Aescin im Bereich von 2·10⁻³ bis 10³ liegt.

10. Zusammensetzung nach dem vorhergehenden Anspruch, **dadurch gekennzeichnet, dass** das Gewichtsverhältnis von Dextransulfat und Aescin im Bereich von 25·10⁻³ bis 200 liegt.

11. Verwendung der Kombination von Dextransulfat und Aescin zur Herstellung einer Zusammensetzung, die dazu vorgesehen ist, präventiv oder kurativ Hautödeme und/oder empfindliche Haut und/oder Prickeln und/oder Kribbeln und/oder Juckreiz und/oder Spannen und/oder Brennen und/oder Erytheme zu behandeln.

12. Kosmetische Verwendung der Kombination von Dextransulfat und Aescin in einer Zusammensetzung, um präventiv oder kurativ Rötungen, Tränensäcke und/oder Augenringe zu behandeln.

13. Kosmetisches Verfahren zur Behandlung von Rötungen, Tränensäcken und/oder Augenringen, das **dadurch gekennzeichnet ist, dass** auf die Haut eine kosmetische Zusammensetzung aufgetragen wird, die zumindest die Kombination von Dextransulfat und Aescin enthält.

## Claims

1. Composition comprising, as active agent, in a physiologically acceptable medium, a combination of dextran sulphate and escin.

2. Composition according to Claim 1, **characterized in that** the dextran sulphate is in the form of a sodium salt.

3. Composition according to either of the preceding claims, **characterized in that** the dextran sulphate has a molecular weight of between 2 × 10³ and 5 × 10⁶.

4. Composition according to the preceding claim, **characterized in that** the dextran sulphate has a molecular weight of between 5 × 10³ and 10⁵.

5. Composition according to any one of the preceding claims, **characterized in that** the dextran sulphate is in an amount by weight of between 0.01% and 5% relative to the total weight of the composition.

6. Composition according to the preceding claim, **characterized in that** the dextran sulphate is in an amount by weight of between 0.05% and 2% relative to the total weight of the composition.

7. Composition according to any one of the preceding claims, **characterized in that** the escin is in an amount by weight of between 0.005% and 5% relative to the total weight of the composition.

8. Composition according to the preceding claim, **characterized in that** the escin is in an amount by weight of between 0.01% and 2% relative to the total weight of the composition.

9. Composition according to any one of the preceding claims, **characterized in that** the weight ratio between the dextran sulphate and the escin is between 2 × 10⁻³ and 10³.

10. Composition according to the preceding claim, **characterized in that** the weight ratio between the dextran sulphate and the escin is between 25 × 10⁻³ and 200.

11. Use of a combination of dextran sulphate and escin for manufacturing a composition intended for preventively or curatively treating skin oedema and/or sensitive skin and/or stinging and/or tingling and/or pruritus and/or tautness and/or heating and/or erythema.

12. Cosmetic use, in a composition of a combination of dextran sulphate and escin for preventively or curatively treating redness, bags and/or dark rings around the eyes.

13. Cosmetic process for treating redness, bags and/or dark rings around the eyes, **characterized in that** a cosmetic composition comprising at least a combination of dextran sulphate and escin is applied to the skin.
